(19) Europäisches Patentamt
European Patent Office
Office européen des brevets

(11) Veröffentlichungsnummer : **0 365 914 B1**

(12) # EUROPÄISCHE PATENTSCHRIFT

(45) Veröffentlichungstag der Patentschrift :
**22.07.92 Patentblatt 92/30**

(21) Anmeldenummer : **89118837.7**

(22) Anmeldetag : **11.10.89**

(51) Int. Cl.$^5$ : **C07C 49/80,** C07C 49/84, C07C 45/00, C07C 45/63, C07C 255/50, C07C 255/54

(54) **Neue Fluor enthaltende und an der CH3-Gruppe gegebenenfalls halogenierte Acetophenone und deren Herstellung aus neuen Fluor enthaltenden Benzonitrilen.**

(30) Priorität : **24.10.88 DE 3836159**

(43) Veröffentlichungstag der Anmeldung :
**02.05.90 Patentblatt 90/18**

(45) Bekanntmachung des Hinweises auf die Patenterteilung :
**22.07.92 Patentblatt 92/30**

(84) Benannte Vertragsstaaten :
**DE FR GB IT**

(56) Entgegenhaltungen :
**EP-A- 0 079 154**
**EP-A- 0 085 265**

(56) Entgegenhaltungen :
**DE-A- 2 550 262**
**FR-A- 1 567 806**
**FR-A- 2 177 093**
**US-A- 3 267 110**

(73) Patentinhaber : **BAYER AG**
**W-5090 Leverkusen 1 Bayerwerk (DE)**

(72) Erfinder : **Kysela, Ernst, Dr.**
**Virchowstrasse 14**
**W-5060 Bergisch Gladbach (DE)**
Erfinder : **Baasner, Bernd, Dr.**
**Hamberger Strasse 27d**
**W-5090 Leverkusen 3 (DE)**
Erfinder : **Schaller, Klaus, Dr.**
**Am Sonnenschein 38**
**W-5600 Wuppertal 1 (DE)**

EP 0 365 914 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Fluor enthaltende und an der $CH_3$-Gruppe gegebenenfalls halogenierte Acetophenone der Formel

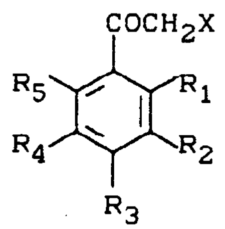

(I),

in der

X für Wasserstoff, Chlor oder Brom steht und

die Reste $R_1$ bis $R_5$ folgende Bedeutung haben,

a) $R_1$ und $R_4$ Fluor, $R_2$ und $R_5$ Chlor und $R_3$ $CF_3$ oder

b) $R_1$, $R_3$ und $R_4$ Fluor, und $R_2$ und $R_5$ Wasserstoff oder

c) $R_1$, $R_4$ und $R_5$ Wasserstoff, $R_2$ Chlor und $R_3$ $CF_3$ oder

d) $R_1$, $R_4$ und $R_5$ Wasserstoff, $R_2$ Chlor und $R_3$ $OCF_3$ oder

e) $R_1$, $R_4$ und $R_5$ Wasserstoff und $R_2$ und $R_3$ $CF_3$ oder

f) $R_1$ Chlor, $R_2$ $CF_3$ und $R_3$, $R_4$ und $R_5$ Wasserstoff oder

g) $R_1$ Chlor, $R_2$, $R_3$ und $R_4$ Wasserstoff und $R_5$ $CF_3$ oder

h) $R_1$ Chlor, $R_2$, $R_4$ und $R_5$ Wasserstoff und $R_3$ $CF_3$.

Fluorhaltige Acetophenone der Formel (I) mit X = Wasserstoff, welche die unter c) bis h) angegebenen Bedeutungen der Substituenten haben, sind bevorzugt. Ganz besonders bevorzugt sind fluorhaltige Acetophenone der Formel (I) mit X = Wasserstoff, welche die unter f) bis h) angegebenen Bedeutungen der Substituenten haben, also 2-Chlor-3-trifluormethyl-acetophenon, 2-Chlor-4-trifluormethyl-acetophenon und 2-Chlor-6-trifluormethyl-acetophenon.

Von den an der $CH_3$-Gruppe halogenierten Acetophenonen der Formel (I) mit X = Chlor oder Brom sind die entsprechenden Verbindungen bevorzugt. Ganz besonders bevorzugt sind dabei 2-Chlor-3-trifluormethyl-phenacyl-bromid und -chlorid, 2-Chlor-4-trifluormethyl-phenacyl-bromid und -chlorid und 2-Chlor-6-trifluormethyl-phenacyl-bromid und -chlorid.

Die vorliegende Erfindung betrifft auch ein Verfahren zur Herstellung von fluorhaltigen Acetophenonen der Formel (I), die gegebenenfalls an der $CH_3$-Gruppe halogeniert sind, das dadurch gekennzeichnet ist, daß man zur Herstellung von Verbindungen der Formel (I) mit X = Wasserstoff ein fluoriertes Benzoesäurederivat der Formel (II)

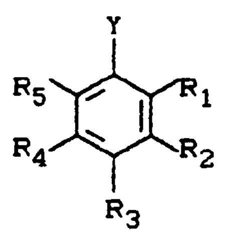

(II),

in der

$R_1$ bis $R_5$ die bei Formel (I) angegebene Bedeutung haben und

Y für eine Nitrilgruppe oder eine Säurehalogenidgruppe

steht,

mit einer zur Einführung von Methylgruppen befähigten magnesiumorganischen Verbindung umsetzt und dann eine Hydrolyse durchführt und zur Herstellung von Verbindungen der Formel (I) mit X = Chlor oder Brom noch anschließend bei -20 bis +80°C mit einem Chlorierungs- oder Bromierungsmittel umsetzt.

Zum Einsatz in das erfindungsgemäße Verfahren sind solche fluorierten Benzoesäurederivate der Formel (II) bevorzugt, bei denen $R_1$ bis $R_5$ die bei Formel (I) unter c) bis h) angegebenen Bedeutungen haben. Besonders bevorzugt sind fluorierte Benzoesäurederivate, bei denen $R_1$ bis $R_5$ die bei Formel (I) unter f) bis h)

angegebenen Bedeutungen haben.

Soweit in Formel (II) Y für eine Säurehalogenidgruppe steht handelt es sich vorzugsweise um eine Säurefluorid-oder Säurechloridgruppe (COF oder COCl), insbesondere um eine Säurefluoridgruppe (COF).

In Formel (II) steht Y vorzugsweise für eine Nitrilgruppe.

Fluorierte Benzoesäurederivate der Formel (II), bei denen Y für eine Säurehalogenidgruppe steht sind bekannt (siehe z.B. DE-OS 3 621 707).

Fluorierte Benzoesäurederivate der Formel (II), bei denen X für eine Nitrilgruppe steht sind teilweise bekannt und teilweise neu. Neu sind insbesondere fluorierte Benzonitrile der Formel (IIa)

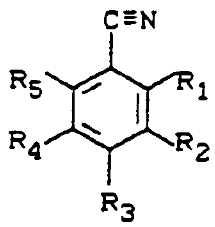

(IIa),

in der

$R_1$, $R_4$ und $R_5$ für Wasserstoff und $R_2$ und $R_3$ für $CF_3$ oder
$R_1$, $R_4$ und $R_5$ für Wasserstoff, $R_2$ für Chlor und $R_3$ für $OCF_3$ oder
$R_2$, $R_3$ und $R_4$ für Wasserstoff, $R_1$ für Chlor und $R_5$ für $CF_3$

stehen.

Die vorliegende Erfindung betrifft deshalb auch solche neuen fluorierten Benzonitrile der Formel (IIa). Herstellungsmöglichkeiten für die neuen fluorierten Benzonitrile sind in den Beispielen 1a), 2a) und 2b) und 5a) angegeben.

Bei den zur Einführung von Methylgruppen befähigten magnesiumorganischen Verbindungen kann es sich beispielsweise um Methylmagnesiumhalogenide, insbesondere um Methylmagnesiumbromid oder Methylmagnesiumiodid handeln, oder um Ethoxymagnesiummalonester.

Letzterer ist beispielsweise zugänglich, indem man Magnesiumdiethoxylat mit Malonester umsetzt und dabei eine Ethoxygruppe des Magnesiumdiethoxylats durch eine Malonestergruppe ersetzt. Bei Ethoxymagnesiummalonester kann man an das C-Atom im Rest Y des fluorierten Benzoesäurederivates der Formel (II) einen Malonesterrest einführen, der dann bei der Hydrolyse durch Decarboxylierung in eine Methylgruppe übergeht.

Bezogen auf 1 Mol fluoriertes Benzoesäurederivat der Formel (II) kann man beispielsweise 0,8 bis 3 Mol der jeweiligen magnesiumorganischen Verbindung einsetzen. Vorzugsweise beträgt diese Menge 1 bis 1,5 Mol. Die magnesiumorganische Verbindung kommt im allgemeinen in gelöster Form zum Einsatz. Geeignete Lösungsmittel sind beispielsweise Ether, insbesondere Diethylether und Tetrahydrofuran. Im allgemeinen stellt man separat eine Lösung der jeweiligen magnesiumorganischen Verbindung her und fügt diese der Verbindung der Formel (II) zu, die ebenfalls in gelöster Form vorliegen kann. Beim Einsatz von Methylmagnesiumhalogeniden kann es vorteilhaft sein eine kleine Menge Katalysator hinzuzufügen, beispielsweise ein Kupfer- oder Eisensalz.

Die Umsetzung des Benzoesäurederivates der Formel (II) mit der magnesiumorganischen Verbindung kann in einem weiten Temperaturbereich durchgeführt werden, beispielsweise zwischen -60 und +100°C. Die nach dieser Umsetzung durchzuführende Hydrolyse kann z.B. durch Eingießen in oder Zufügen von Wasser und mehrstündiges Halten bei einer Temperatur im Bereich von -10 bis +40°C bewerkstelligt werden. Vorzugsweise setzt man Säure zu, beispielsweise Essig-, Salz- oder Schwefelsäure. Die Durchführung der Hydrolyse in Gegenwart einer starken Säure ist insbesondere dann vorteilhaft, wenn als magnesiumorganische Verbindung Ethoxymagnesiummalonester eingesetzt worden ist.

Das nach der Hydrolyse vorliegende Reaktionsgemisch kann man beispielsweise aufarbeiten indem man die organische Phase daraus abtrennt und diese fraktioniert destilliert.

Durch die Umsetzung eines Benzosäurederivats der Formel (II) mit einer zur Einführung von Methylgruppen befähigten magnesium-organischen Verbindung mit nachfolgender Hydrolyse erhält man Fluor enthaltende Acetophenone der Formel (I) mit X = Wasserstoff. Aus diesen kann man an der $CH_3$-Gruppe halogenierte Acetophenone der Formel (I) mit X = Chlor oder Brom erhalten, wenn man sie bei -20 bis +80°C mit einem Chlorierungs- oderBromierungsmittel umsetzt. Ein geeignetes Chlorierungsmittel ist beispielsweise Sulfurylchlorid ($SO_2Cl_2$); als Bromierungsmittel ist beispielsweise elementares Brom geeignet.

Im allgemeinen setzt man das Chlorierungs- oder Bromierungsmittel in der stöchiometrisch erforderlichen

Menge oder im Überschuß ein, beispielsweise 1 bis 1,2 Mol pro Mol Edukt. Geeignete Reaktionstemperaturen sind solche im Bereich -20 bis +80°C, insbesondere solche von 0 bis 40°C.

Man kann die Chlorierung bzw. Bromierung in Anwesenheit oder Abwesenheit von Lösungsmitteln durchführen. Vorzugsweise arbeitet man in Gegenwart inerter organischer Lösungsmittel, beispielsweise Methylenchlorid oder Eisessig. Ebenso ist es nicht zwingend nötig, aber im allgemeinen vorteilhaft, in Gegenwart katalytischer Mengen einer starken, konzentrierten Mineralsäure zu arbeiten. Beispielsweise sind hier Schwefelsäure oder Salzsäure geeignet.

Das Ende der Chlorierung bzw. Bromierung ist am Aufhören der Gasentwicklung (Chlor- oder Bromwasserstoff) zu erkennen. Man kann dann das Reaktionsgemisch beispielsweise so aufarbeiten, daß man es mit Wasser oder Eiswasser vermischt, mit einem organischen Lösungsmittel ausschüttelt, den organischen Extrakt einengt und den Rückstand destilliert. Gegebenenfalls kann eine weitere Reinigung durchgeführt werden, z.B. durch Umkristallisation, Destillation oder Chromatographie.

Die neuen, Fluor enthaltenden Acetophenone der Formel (I) mit X = Chlor oder Brom lassen sich durch Umsetzung mit einem Thioharnstoffderivat des Typs

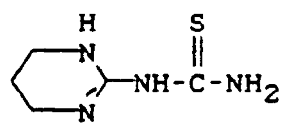

in substituierte Aminothiazole des Typs

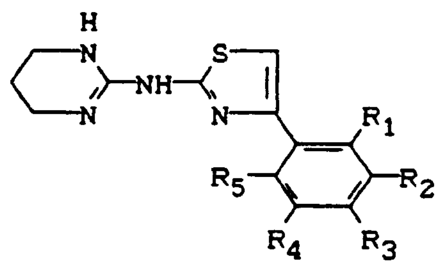

überführen,
bei denen es sich um Verbindungen handelt, die eine gute Wirksamkeit gegen Schädlinge, insbesondere pflanzenschädigende Pilze besitzen.

Substituierte Aminothiazole des genannten Typs, deren Herstellung und Verwendung sind Gegenstand einer separaten eigenen Patentanmeldung.

Es ist als überraschend anzusehen, daß die erfindungsgemäßen fluorierten Acetophenone der Formel (I) auf die beschriebene Weise in guten Ausbeuten zugänglich sind, denn außer der gewünschten Reaktion waren Reaktionen mit den aktivierten, am Kern gebundenen Halogenatomen und Hydrolyse der am Kern gebundenen $CF_3$-Gruppen zu erwarten.

Beispiele

Beispiel 1

a) 3,4-Bistrifluormethyl-benzonitril

229 g (1 Mol) 3,4-Bistrifluormethyl-anilin wurden in einer Lösung von 350 g konzentrierter Schwefelsäure in 1,25 l Wasser mit 70 g Natriumnitrit in 140 ml Wasser diazotiert. Nachdem die Diazoniumsalzlösung nitritfrei war wurde sie in eine auf 100°C erwärmte Lösung bestehend aus 550 ml Wasser, 210 g Natriumcyanid, 10 g Kupfer(I)-cyanid, 500 g Natriumhydrogencarbonat und 9 g Nickelsulfat x 7 $H_2O$ getropft. Aus dem Reaktionsgemisch wurde das Produkt durch Wasserdampfdestillation isoliert und anschließend nochmals destilliert. Es wurden 152 g Produkt mit einem Siedepunkt bei 14 mbar von 85°C und einem Schmelzpunkt von 71 bis 72°C erhalten. Das entspricht einer Ausbeute von 63 % der Theorie.

b) 3,4-Bistrifluormethyl-acetophenon

119,5 g (0,5 Mol) 3,4-Bistrifluormethyl-benzonitril wurden mit 166 g (1 Mol) Methylmagnesiumiodid in 750 ml Benzol 3 Stunden am Rückfluß gekocht. Nach dem Abkühlen auf 0° wurden 500 ml 6 n wäßrige Salzsäure zufließen gelassen und weitere 6 Stunden am Rückfluß gekocht. Danach wurde das Gemisch abgekühlt, die organische Phase abgetrennt und destilliert. Es wurden 123 g Produkt mit einem Siedepunkt bei 0,3 mbar von 71 bis 73°C erhalten. Das entspricht einer Ausbeute von 48 % der Theorie.

Beispiel 2

a) 3-Chlor-4-trifluormethoxy-benzamid

242,5 g (1 Mol) 3-Chlor-4-trifluormethoxy-benzoylfluorid wurden in 500 ml 25 gew.-%ige wäßrige Ammoniaklösung unter Eiskühlung eintropfen gelassen, danach 30 Minuten nachgerührt und der ausgefallene Niederschlag abgesaugt. Es wurden 227 g Produkt mit einem Schmelzpunkt von 98°C erhalten. Das entspricht einer Rohausbeute von 95 % der Theorie.

b) 3-Chlor-4-trifluormethoxy-benzonitril

239,5 g (1 Mol) 3-Chlor-4-trifluormethoxy-benzamid wurden mit 750 ml $SOCl_2$ versetzt und die Mischung langsam (entsprechend der Gasentwicklung) bis auf 85°C erwärmt. Anschließend wurde das Gemisch fraktioniert destilliert und 189 g Produkt mit einem Siedepunkt bei 13 mbar von 96°C und einem Schmelzpunkt von 38 bis 40°C erhalten. Das entspricht einer Ausbeute von 85 % der Theorie.

c) 3-Chlor-4-trifluormethoxy-acetophenon

Analog Beispiel 1b) wurden 221,5 g (1 Mol) 3-Chlor-4-trifluormethoxy-benzonitril mit Methylmagnesiumiodid umgesetzt und das Reaktionsgemisch entsprechend aufgearbeitet. Es wurden 105,4 g Produkt mit einem Siedepunkt bei 0,1 mbar von 98 bis 99°C erhalten. Das entspricht einer Ausbeute von 44 % der Theorie.

Beispiel 3

3-Chlor-4-trifluormethyl-acetophenon

226 g (1 Mol) 3-Chlor-4-trifluormethyl-benzoylfluorid wurden in 500 ml Diethylether vorgelegt und nach Zusatz von 3 g $FeCl_3$ eine aus 95 g (1 Mol) Methylbromid und 24,3 g Magnesium in 250 ml Diethylether hergestellte Grignard-Lösung bei -60°C Innentemperatur innerhalb von 4 Stunden zugetropft. Es wurde noch 24 Stunden bei -60°C gehalten, dann auf 25°C erwärmt. Danach wurde das Reaktionsgemisch in Wasser geschüttet, die organische Phase abgetrennt und fraktioniert destilliert. Es wurden 47,5 g Produkt mit einem Siedepunkt bei 0,2 mbar von 84 bis 87°C erhalten. Das entspricht einer Ausbeute von 20 % der Theorie.

Beispiel 4

2-Chlor-4-trifluormethyl-acetophenon

81 g (0,358 Mol) 2-Chlor-4-trifluormethyl-benzoylchlorid wurden analog Beispiel 3 mit Methylmagnesiumbromid umgesetzt und das Reaktionsgemisch entsprechend aufgearbeitet. Es wurden 35,2 g Produkt mit einem Siedepunkt bei 10 mbar von 80 bis 81°C erhalten. Das entspricht einer Ausbeute von 44 % der Theorie.

Beispiel 5

a) 2-Chlor-6-trifluormethyl-benzonitril

255 g (1 Mol) 2-Chlor-6-trichlormethyl-benzonitril und 250 g wasserfreier Fluorwasserstoff wurden in einem Autoklaven 4 Stunden lang auf 140°C erwärmt. Der entstehende Chlorwasserstoff wurde bei 25 bar kontinuierlich entweichen gelassen. Danach wurde überschüssiger Fluorwasserstoff abgezogen, der Reaktionsrückstand destilliert, das Destillat im Siedebereich von 80 bis 142°C bei 15 mbar aufgefangen (180 g), dieses mit 55 g Antimontrifluorid versetzt und auf 90°C erwärmt. In die 90°C warme Reaktionsmischung

wurde eine kleine Menge Chlor eingeleitet um das Antimontrifluorid zu aktivieren. Danach wurde noch 1 Stunde auf 135°C erwärmt. Zur Aufarbeitung wurde das Reaktionsgemisch in Wasser geschüttet, die organische Phase abgetrennt und destilliert. Es wurden 138 g Produkt mit einem Siedepunkt bei 13 mbar von 112 bis 113°C und einem Schmelzpunkt von 45 bis 47°C erhalten. Das entspricht einer Ausbeute von 67 % der Theorie.

b) 2-Chlor-6-trifluormethyl-acetophenon

154 g (0,75 Mol) 2-Chlor-6-trifluormethyl-benzonitril wurden in 375 ml Diethylether vorgelegt und nach Zusatz von 1 g CuCl eine aus 24,3 g Magnesium und 95 g (1 Mol) Methylbromid in 250 ml Diethylether hergestellte Grignard-Lösung bei 28 bis 30°C innerhalb von 3 Stunden zugetropft. Es wurde noch 5 Stunden bei 28 bis 30°C nachgerührt. Dann wurde das Reaktionsgemisch in Wasser gegossen, die organische Phase abgetrennt und destilliert. Es wurden 114 g Produkt mit einem Siedepunkt bei 0,3 mbar von 65 bis 66°C erhalten. Das entspricht einer Ausbeute von 68 % der Theorie.

Beispiel 6

2-Chlor-3-trifluormethyl-acetophenon

193 g (0,94 Mol) 2-Chlor-3-trifluormethyl-benzonitril wurden analog Beispiel 5 mit Methylmagnesiumbromid umgesetzt und das Reaktionsgemisch entsprechend aufgearbeitet. Es wurden 94 g Produkt mit einem Siedepunkt bei 8 mbar von 90 bis 91°C erhalten. Das entspricht einer Ausbeute von 45 % der Theorie.

Beispiel 7

2,4,5-Trifluor-acetophenon

194,5 g (1 Mol) 2,4,5-Trifluor-benzoylchlorid wurden in 100 ml Diethylether vorgelegt und zum Sieden am Rückfluß erwärmt. Dann wurden 1,1 Mol Ethoxymagnesiummalonester gelöst in 100 ml Ethanol und 125 ml Diethylether innerhalb von 30 Minuten zutropfen gelassen und 1 Stunde bei Rückfluß nachgerührt. Nach dem Abkühlen wurde das Reaktionsgemisch in 500 ml Eiswasser eingerührt, mit konzentrierter Schwefelsäure auf einen pH-Wert von 1 eingestellt und das organische Material (345 g) abgetrennt. Dieses organische Material wurde in 300 ml Essigsäure gelöst und nach dem Zusatz von 37,5 ml konzentrierter Schwefelsäure bis zum Ende der $CO_2$-Entwicklung am Rückfluß gekocht, was 6 Stunden dauerte. Danach wurde das Reaktionsgemisch abgekühlt, auf Wasser gegossen, die organische Phase abgetrennt und destilliert. Es wurden 83 g Produkt mit einem Siedepunkt bei 10 mbar von 63 bis 64°C erhalten. Das entspricht einer Ausbeute von 47 % der Theorie.

Beispiele 8 bis 14

Allgemeine Arbeitsvorschrift:

Zu 0,125 Mol einer Verbindung der Formel (I) mit X = Wasserstoff wurden in 250 ml Eisessig, dem 1,25 ml konzentrierte Salzsäure zugesetzt worden waren, bei Raumtemperatur (22°C) im Verlauf von 2 Stunden 22,4 g (0,14 Mol) Brom gelöst in 50 ml Eisessig zugetropft. Es wurde weitere 2 Stunden bei Raumtemperatur nachgerührt. Dann wurde das Reaktionsgemisch auf 1 l Eiswasser gegossen, die organische Phase abgetrennt, die wäßrige Phase zweimal mit je 100 ml Dichlormethan extrahiert, die vereinigten organischen Phasen zweimal mit je 150 ml Wasser gewaschen und über Magnesiumsulfat getrocknet. Danach wurde im Wasserstrahlvakuum das Lösungsmittel abgezogen. Die Einzelheiten der durchgeführten Umsetzungen sind aus Tabelle 1 ersichtlich, ebenso die durch Aufnahme des $^1H$-NMR-Spektrums (in $CDCl_3$ mit Tetramethylsilan als internen Standard) vorgenommene Charakterisierung der erhaltenen Produkte (angegeben ist jeweils der $\delta$-Wert in ppm für die Protonen der $-CH_2$-X-Gruppe).

**Tabelle 1**

| Beispiel Nr. | Einsatzprodukt erhalten gemäß Beispiel | Reaktionsprodukt der Formel (I) mit X = Brom (nicht genannte Substituenten sind Wasserstoff) | Ausbeute [% d.Th] | Charakterisierung |
|---|---|---|---|---|
| 8 | 1b | $R_2 = R_3 = CF_3$ | 83,8 | 5,30 |
| 9 | 2c | $R_2 = Cl$, $R_3 = OCF_3$ | 76,9 | 5,33 |
| 10 | 3 | $R_2 = Cl$, $R_3 = CF_3$ | 81,2 | 5,31 |
| 11 | 4 | $R_1 = Cl$, $R_3 = CF_3$ | 78,8 | 5,31 |
| 12 | 5b | $R_1 = Cl$, $R_5 = CF_3$ | 71 | 5,34 |
| 13 | 6 | $R_1 = Cl$, $R_2 = CF_3$ | 89 | 5,36 |
| 14 | 8 | $R_1 = R_2 = R_4 = R_5 = F$; $R_3 = CF_3$ | 94,3 | 5,20 |

Beispiel 15

Zu 48 g (0,275 Mol) 2,4,5-Trifluoracetophenon (erhalten gemäß Beispiel 6) gelöst in 400 ml Dichlormethan wurden bei Raumtemperatur (22°C) 40,8 g (0,3 Mol) Sulfurylchlorid getropft und bis zum Ende der Chlorwasserstoffentwicklung (rund 2 Stunden) nachgerührt. Dann wurden dem Reaktionsgemisch 600 ml Wasser zugegeben, die organische Phase abgetrennt, diese mit Natriumhydrogencarbonat-Lösung neutral gewaschen, über Magnesiumsulfat getrocknet und im Wasserstrahlvakuum das Lösungsmittel abgezogen. Es wurden als öliger Rückstand 48,6 g 2,4,5-Trifluor-phenacylchlorid erhalten, das entspricht 84,5 % der Theorie. Die wie bei den Beispielen 9 bis 15 vorgenommene Charakterisierung des Produktes lieferte einen δ-Wert von 5.29 ppm.

## Patentansprüche

1. Fluor enthaltende und an der $CH_3$-Gruppe gegebenenfalls halogenierte Acetophenone der Formel

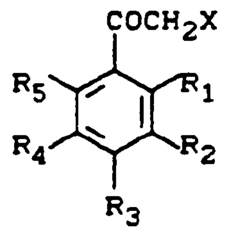

(I),

in der

X für Wasserstoff, Chlor oder Brom steht und

die Reste $R_1$ bis $R_5$ folgende Bedeutung haben,

a) $R_1$ und $R_4$ Fluor, $R_2$ und $R_5$ Chlor und $R_3$ $CF_3$ oder

b) $R_1$, $R_3$ und $R_4$ Fluor, und $R_2$ und $R_5$ Wasserstoff oder

c) $R_1$, $R_4$ und $R_5$ Wasserstoff, $R_2$ Chlor und $R_3$ $CF_3$ oder

d) $R_1$, $R_4$ und $R_5$ Wasserstoff, $R_2$ Chlor und $R_3$ $OCF_3$ oder

e) $R_1$, $R_4$ und $R_5$ Wasserstoff und $R_2$ und $R_3$ $CF_3$ oder

f) $R_1$ Chlor, $R_2$ $CF_3$ und $R_3$, $R_4$ und $R_5$ Wasserstoff oder

g) $R_1$ Chlor, $R_2$, $R_3$ und $R_4$ Wasserstoff und $R_5$ $CF_3$ oder

h) $R_1$ Chlor, $R_2$, $R_4$ und $R_5$ Wasserstoff und $R_3$ $CF_3$.

2. Fluor enthaltende Acetophenone gemäß Anspruch 1, dadurch gekennzeichnet, daß es sich um 2-Chlor-3-trifluormethyl-acetophenon, 2-Chlor-4-trifluormethyl-acetophenon, 2-Chlor-6-trifluormethyl-acetophenon, 2-Chlor-3-trifluormethyl-phenacyl-bromid und -chlorid, 2-Chlor-4-trifluormethylphenacylbromid und -chlorid und 2-Chlor-6-trifluormethylphenacyl-bromid und -chlorid handelt.

3. Verfahren zur Herstellung von Fluor enthaltenden Acetophenonen der Formel (I), die gegebenenfalls an der $CH_3$-Gruppe halogeniert sind, dadurch gekennzeichnet, daß man zur Herstellung von Verbindungen der Formel (I) mit X = Wasserstoff ein fluoriertes Benzoesäurederivat der Formel (II)

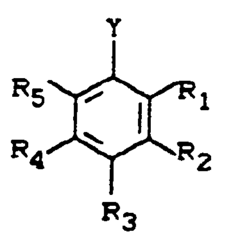

(II),

in der

$R_1$ bis $R_5$ die bei Formel (I) angegebene Bedeutung haben und

Y für eine Nitrilgruppe oder eine Säurehalogenidgruppe steht,

mit einer zur Einführung von Methylgruppen befähigten magnesiumorganischen Verbindung umsetzt und anschließend eine Hydrolyse durchführt und zur Herstellung von Verbindungen der Formel (I) mit X = Chlor oder Brom noch anschließend bei -20 bis +80°C mit einem Chlorierungs- oder Bromierungsmittel umsetzt.

4. Verfahren nach Anspruch 3, dadurch gekennzeichnet, daß als zur Einführung von Methylgruppen befähigte magnesiumorganische Verbindungen Methylmagnesiumhalogenide oder Ethoxymagnesiummalonester verwendet werden.

5. Verfahren nach Ansprüchen 3 und 4, dadurch gekennzeichnet, daß man pro Mol fluoriertes Benzoesäurederivat der Formel (II) 0,8 bis 3 Mol der jeweiligen magnesiumorganischen Verbindung einsetzt.

6. Verfahren nach Ansprüchen 3 bis 5, dadurch gekennzeichnet, daß man die Umsetzung mit der magnesiumorganischen Verbindung in Gegenwart eines Ethers als Lösungsmittel durchführt.

7. Verfahren nach Ansprüchen 3 bis 6, dadurch gekennzeichnet, daß man die Umsetzung mit der magnesiumorganischen Verbindung bei Temperaturen im Bereich -60 bis +100°C durchführt.

8. Verfahren nach Ansprüchen 3 bis 7, dadurch gekennzeichnet, daß man die Umsetzung mit einem Chlorierungs- oder Bromierungsmittel mit Sulfurylchlorid oder elementaren Brom durchführt.

9. Verfahren nach Anspruch 8, dadurch gekennzeichnet, daß man die Umsetzung bei 0 bis 40°C durchführt.

10. Fluorierte Benzonitrile der Formel (IIa)

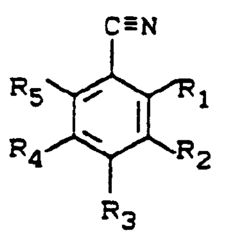

(IIa),

in der

$R_1$, $R_4$ und $R_5$ für Wasserstoff und $R_2$ und $R_3$ für $CF_3$ oder
$R_1$, $R_4$ und $R_5$ für Wasserstoff, $R_2$ für Chlor und $R_3$ für $OCF_3$ oder
$R_2$, $R_3$ und $R_4$ für Wasserstoff, $R_1$ für Chlor und $R_5$ für $CF_3$

stehen.

## Claims

1. Fluorine-containing acetophenones, optionally halogenated on the $CH_3$ group, of the formula

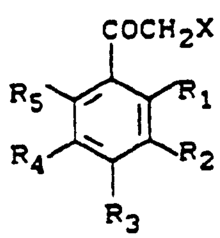

(I),

in which

X represents hydrogen, chlorine or bromine and

the radicals $R_1$ to $R_5$ have the following meaning,

a ) $R_1$ and $R_4$ denote fluorine, $R_2$ and $R_5$ denote chlorine and $R_3$ denotes $CF_3$, or
b) $R_1$, $R_3$ and $R_4$ denote fluorine and $R_2$ and $R_5$ denote hydrogen, or
c) $R_1$, $R_4$ and $R_5$ denote hydrogen, $R_2$ denotes chlorine and $R_3$ denotes $CF_3$, or
d) $R_1$, $R_4$ and $R_5$ denote hydrogen, $R_2$ denotes chlorine and $R_3$ denotes $OCF_3$, or
e ) $R_1$, $R_4$ and $R_5$ denote hydrogen and $R_2$ and $R_3$ denote $CF_3$, or
f) $R_1$ denotes chlorine, $R_2$ denotes $CF_3$ and $R_3$, $R_4$ and $R_5$ denote hydrogen, or
g) $R_1$ denotes chlorine, $R_2$, $R_3$ and $R_4$ denote hydrogen and $R_5$ denotes $CF_3$, or
h) $R_1$ denotes chlorine, $R_2$, $R_4$ and $R_5$ denote hydrogen and $R_3$ denotes $CF_3$.

2. Fluorine-containing acetophenones according to Claim 1, characterized in that they are 2-chloro-3-trifluoromethyl-acetophenone, 2-chloro-4-trifluoromethyl-acetophenone, 2-chloro-6-trifluoromethyl-acetophenone, 2-chloro-3-trifluoromethyl-phenacyl bromide and chloride, 2-chloro-4-trifluoromethylphenacyl bromide and

chloride and 2-chloro-6-trifluoromethylphenacyl bromide and chloride.

3. Process for the preparation of fluorine-containing acetophenones of the formula (I), which are optionally halogenated on the $CH_3$ group, characterized in that, for the preparation of compounds of the formula (I) in which X = hydrogen, a fluorinated benzoic acid derivative of the formula (II)

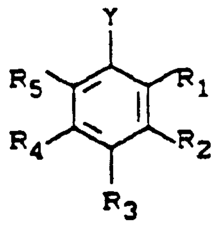

(II),

in which

$R_1$ to $R_5$ have the meaning indicated in formula (I) and

Y represents a nitrile group or an acid halide group,

is reacted with an organomagnesium compound capable of introducing methyl groups and a hydrolysis is then carried out and, for the preparation of compounds of the formula (I) in which X = chlorine or bromine, the product is further subsequently reacted at -20 to +80°C with a chlorinating or brominating agent.

4. Process according to Claim 3, characterized in that methylmagnesium halides or ethoxymagnesium ethyl malonates are used as organomagnesium compounds capable of introducing methyl groups.

5. Process according to Claims 3 and 4, characterized in that 0.8 to 3 moles of the respective organomagnesium compound are employed per mole of fluorinated benzoic acid derivative of the formula (II).

6. Process according to Claims 3 to 5, characterized in that the reaction with the organomagnesium compound is carried out in the presence of an ether as the solvent.

7. Process according to Claims 3 to 6, characterized in that the reaction with the organomagnesium compound is carried out at temperatures in the range from - 60 to +100°C.

8. Process according to Claims 3 to 7, characterized in that the reaction with a chlorinating or brominating agent is carried out using sulphuryl chloride or elemental bromine.

9. Process according to Claim 8, characterized in that the reaction is carried out at 0 to 40°C.

10. Fluorinated benzonitriles of the formula (IIa)

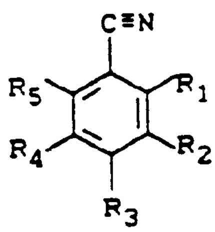

(IIa),

in which

$R_1$, $R_4$ and $R_5$ represent hydrogen and $R_2$ and $R_3$ represent $CF_3$ or

$R_1$, $R_4$ and $R_5$ represent hydrogen, $R_2$ represents chlorine and $R_3$ represents $OCF_3$ or

$R_2$, $R_3$ and $R_4$ represent hydrogen, $R_1$ represents chlorine and $R_5$ represents $CF_3$.

**Revendications**

1. Acétophénones contenant du fluor et pouvant être halogénées sur le groupe $CH_3$, de formule

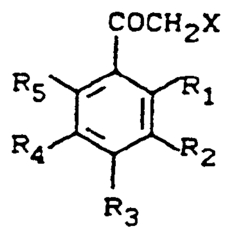

(I),

dans laquelle

X représente l'hydrogène, le chlore ou le brome et

les restes $R_1$ à $R_5$ ont les définitions suivantes

a) $R_1$ et $R_4$ sont du fluor, $R_2$ et $R_5$ sont du chlore et $R_3$ est un groupe $CF_3$, ou bien

b) $R_1$, $R_3$ et $R_4$ sont du fluor et $R_2$ et $R_5$ sont de l'hydrogène, ou bien

c) $R_1$, $R_4$ et $R_5$ sont de l'hydrogène, $R_2$ est du chlore et $R_3$ est un groupe $CF_3$, ou bien

d) $R_1$, $R_4$ et $R_5$ sont de l'hydrogène, $R_2$ est du chlore et $R_3$ est un groupe $OCF_3$, ou bien

e ) $R_1$, $R_4$ et $R_5$ sont de l'hydrogène et $R_2$ et $R_3$ sont des groupes $CF_3$, ou bien

f) $R_1$ est du chlore, $R_2$ est un groupe $CF_3$ et $R_3$, $R_4$ et $R_5$ sont de l'hydrogène, ou bien

g) $R_1$ est du chlore, $R_2$, $R_3$ et $R_4$ sont de l'hydrogène et $R_5$ est un groupe $CF_3$, ou bien

h ) $R_1$ est du chlore, $R_2$, $R_4$ et $R_5$ sont de l'hydrogène et $R_3$ est un groupe $CF_3$.

2. Acétophénones contenant du fluor suivant la revendication 1, caractérisées en ce qu'il s'agit de la 2-chloro-3-trifluorométhyl-acétophénone, de la 2-chloro-4-trifluorométhyl-acétophénone, de la 2-chloro-6-trifluorométhyl-acétophénone, du bromure et du chlorure de 2-chloro-3-trifluorométhyl-phénacyle, du bromure et du chlorure de 2-chloro-4-trifluorométhylphénacyle et du bromure et du chlorure de 2-chloro-6-trifluorométhylphénacyle.

3. Procédé de production d'acétophénones contenant du fluor de formule (I), qui sont éventuellement halogénées sur le groupe $CH_3$, caractérisé en ce qu'on fait réagir, pour l'obtention de composés de formule (I) dans laquelle X est l'hydrogène, un dérivé fluoré d'acide benzoïque de formule (II)

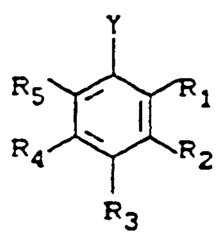

(II),

dans laquelle

$R_1$ à $R_5$ ont la définition indiquée pour la formule (I) et

Y représente un groupe nitrile ou un groupe halogénure d'acide,

avec un composé organomagnèsien apte à introduire des groupes méthyle puis on conduit une hydrolyse et, pour l'obtention de composés de formule (I) dans laquelle X est le chlore ou le brome, on poursuit encore la réaction entre -20 et +80°C avec un agent de chloration ou de bromation.

4. Procédé suivant la revendication 3, caractérisé en ce qu'on utilise comme composés organomagnésiens aptes à introduire des groupes méthyle, des halogénures de méthylmagnésium ou des esters maloniques d'éthoxymagnésium.

5. Procédé suivant les revendications 3 et 4, caractérisé en ce qu'on utilise, par mole de dérivé fluoré d'acide benzoïque de formule (II), 0,8 à 3 moles du composé organomagnésien concerné.

6. Procédé suivant les revendications 3 à 5, caractérisé en ce qu'on conduit la réaction avec le composé organomagnésien en présence d'un éther utilisé comme solvant.

7. Procédé suivant les revendications 3 à 6, caractérisé en ce qu'on conduit la réaction avec le composé organomagnésien à des températures comprises dans l'intervalle de -60 à +100°C.

8. Procédé suivant les revendications 3 à 7, caractérisé en ce qu'on conduit la réaction avec l'agent de chloration ou de bromation, avec du chlorure de sulfuryle ou du brome élémentaire.

9. Procédé suivant la revendication 8, caractérisé en ce qu'on conduit la réaction à une température de 0 à 40°C.

10. Benzonitriles fluorés de formule (IIa)

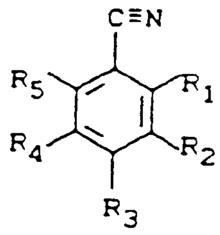

(IIa)

dans laquelle

$R_1$, $R_4$ et $R_5$ sont de l'hydrogène et $R_2$ et $R_3$ sont des groupes $CF_3$, ou bien

$R_1$, $R_4$ et $R_5$ sont de l'hydrogène, $R_2$ est du chlore et $R_3$ est un groupe $OCF_3$, ou bien

$R_2$, $R_3$ et $R_4$ sont de l'hydrogène, $R_1$ est du chlore et $R_5$ est un groupe $CF_3$.